# EUROPEAN PATENT APPLICATION

(11) **EP 4 579 094 A1**
(43) Date of publication of application: **02.07.2025**
(21) Application number: 24222606.6
(22) Date of filing: 20.12.2024
(51) Int. Cl.: F16B 2/12, A61M 1/16, A61M 1/36, A61M 60/845, F16B 2/18, F16M 11/04

(54) **VERTICAL QUICK CLAMPING FIXING DEVICE**

(30) Priority: 25.12.2023 CN 202311794657
(71) Applicant: ChinaBridge (Shenzen) Medical Technology Co., Ltd., Shenzhen, Guangdong 518102 (CN)
(72) Inventor: LI, Yijiang, Shenzhen Guangdong, 518102 (CN)
(74) Representative: Pfenning, Meinig & Partner mbB

(57) **Abstract**

A vertical quick clamping fixing device includes: the fixing seat comprises a mounting groove with an upper end opening; the locking block comprises a mounting part, a connecting part and a fixing part, wherein the mounting part is used for being fixedly mounted with a piece to be fixed, and the connecting part is used for connecting the mounting part and the fixing part; the connecting part can extend into the mounting groove from the opening so as to place the fixing part in the fixing seat; the edge of fixed part equidistant is equipped with the spacing groove just the both sides of spacing groove have the guide surface, the fixing base still includes elastic locating part, elastic locating part has elasticity and can stretch out and draw back, so that elastic locating part butt all the time is in the edge of fixed part, just elastic locating part can follow the guide surface that moves in or moves out the spacing groove. The vertical quick clamping and fixing device in the embodiment can adjust the position of the pump body after fixing the pump body.

## Description

### TECHNICAL FIELD

The embodiments of the application belong to the field of medical instruments, and particularly relate to a vertical rapid clamping and fixing device.

### BACKGROUND

In emergency treatment of severe patients with severe cardiopulmonary failure, sustained in vitro respiratory and circulatory support is provided to the patient by means of extracorporeal membrane oxygenation (ECMO) to fight more precious time for emergency treatment. The pump driving device is used as a core component and is combined with the pump body to drive the whole blood to circulate. Most of pump driving devices used in ECMO in the market at present have fixed installation angles or pump driving is integrated with a host, and the installation angles cannot be adjusted in a rotating way. Some clamping devices with adjustable rotation angles, such as patent CN1 15569258A, have the following drawbacks: the knob can be rotated only after the finger rotates for a plurality of circles, and the operation is complex; when the pump is driven and clamped, the two are matched in a T shape, and if the open groove is small, the situation that alignment is difficult exists. When only the pump is driven to be put in on one side, the condition that the lock can be continuously locked down also exists, and the safety is not high; the finger is rotated and screwed during locking, which is time-consuming and labor-consuming; during locking, as the locking action is finger tightening, the tightening degree is subjective, and whether the pump drive is locked cannot be intuitively felt. The existing pump body fixing equipment is difficult to simply and conveniently adjust the position of the pump body after fixing the pump body.

### SUMMARY

In order to solve the problem that the pump body position cannot be simply and conveniently adjusted after the pump body installation device in the prior art is installed, the invention provides a vertical quick clamping and fixing device, which comprises:
the fixing seat comprises a mounting groove with an upper end opening;
the locking block comprises a mounting part, a connecting part and a fixing part, wherein the mounting part is used for being fixedly mounted with a piece to be fixed, and the connecting part is used for connecting the mounting part and the fixing part;
the connecting part can extend into the mounting groove from the opening so as to place the fixing part in the fixing seat, and the connecting part can also rotate in the mounting groove;
the edge of fixed part equidistant is equipped with the spacing groove just the both sides of spacing groove have the guide surface, the fixing base still includes elastic locating part, elastic locating part is equipped with elasticity and can stretch out and draw back, so that elastic locating part butt all the time is in the edge of fixed part, just elastic locating part can follow the guide surface moves in or moves out the spacing groove.

As a preferred embodiment of the application, the locking mechanism further comprises a locking mechanism, wherein the locking mechanism comprises a rotating piece and a limiting piece which are mutually abutted, the rotating piece is rotatably arranged on the fixing seat, the limiting piece is movably arranged on the fixing seat, and the rotating piece can enable the limiting piece to move along the axial direction of the rotating piece;
the fixing part can be extruded by the limiting piece so that the locking block is fixed on the fixing seat.

As a preferred embodiment of the present application, the rotating member includes a rotating shaft and a rotating cam, where the rotating cam is fixedly disposed at one end of the rotating shaft near the fixing seat, and a protrusion is disposed on a side of the rotating cam facing away from the rotating shaft; the limiting piece is provided with an arc-shaped groove matched with the protrusion on one side facing the rotating piece, and the height of the arc-shaped groove is set from high to low along the circumferential direction of the rotating piece.

As a preferred embodiment of the present application, the arc-shaped groove is provided with a first section and a second section, wherein the inclination angle of the groove bottom of the first section is larger than that of the second section.

As a preferred embodiment of the present application, the rotating member further includes a grip member, and the grip member is driven to rotate the rotating shaft.

As a preferred embodiment of the present application, a first wave surface is disposed on a side of the limiting member facing the fixing portion, a second wave surface is disposed on a side of the fixing portion facing the limiting member, and the first wave surface and the second wave surface can be engaged.

As a preferred embodiment of the application, the limit groove and the guide surface are connected end to form a third waveform surface, the trough of the second waveform surface corresponds to the crest of the third waveform surface, and the crest of the second waveform surface corresponds to the trough of the third waveform surface.

As a preferred embodiment of the present application, the device further includes a first elastic member, where the first elastic member is connected to the fixing base and the rotating cam, respectively, so that the rotating cam can be reset after being rotated.

As a preferred embodiment of the present application, the fixing device further includes a second elastic member, where the second elastic member is disposed between the fixing base and the limiting member, and the second elastic member can enable the limiting member to be far away from the fixing portion.

As a preferred embodiment of the present application, the connection part has a cylindrical shape so that the locking block can be rotated.

As a preferred embodiment of the present application, the device further comprises a dust-proof structure, wherein the dust-proof structure can be hinged to the fixing seat and can cover the opening.
Compared with the prior art, the vertical rapid clamping and fixing device provided by the invention has the advantages that the fixing seat is fixed, and the mounting part of the locking block is fixedly connected with the pump body in advance. When the pump body needs to be installed or replaced, the locking block with the pump body is directly inserted into the installation groove from the opening. The elastic positioning part is abutted against the edge of the fixing part, and the elastic positioning part can be always positioned in the limiting groove due to the fact that the edge is provided with a natural guiding surface and the limiting groove. When the position of the pump body needs to be adjusted, the pump body is directly rotated to drive the fixing part to rotate. The elastic positioning part has elasticity and can stretch out and draw back, so that the elastic positioning part can move from one limiting groove to the other limiting groove, and the purpose of repositioning the pump body is realized.

### BRIEF DESCRIPTION OF THE DRAWINGS

The accompanying drawings, which are included to provide a further understanding of the application and are incorporated in and constitute a part of this application, illustrate embodiments of the application and together with the description serve to explain the application and do not constitute an undue limitation to the application. Some specific embodiments of the present application will be described in detail hereinafter by way of example and not by way of limitation with reference to the accompanying drawings. The same reference numbers in the drawings denote the same or similar parts or portions, and it will be understood by those skilled in the art that the drawings are not necessarily drawn to scale, in which:
FIG. 1 is a schematic view showing a structure of a vertical quick clamping fixture and a pump body in a use state in an embodiment of the present invention;
FIG. 2 shows a schematic top view of a vertical quick clamp fixture in accordance with an embodiment of the present invention;
FIG. 3 shows an exploded view of a vertical quick clamp fixture in an embodiment of the invention;

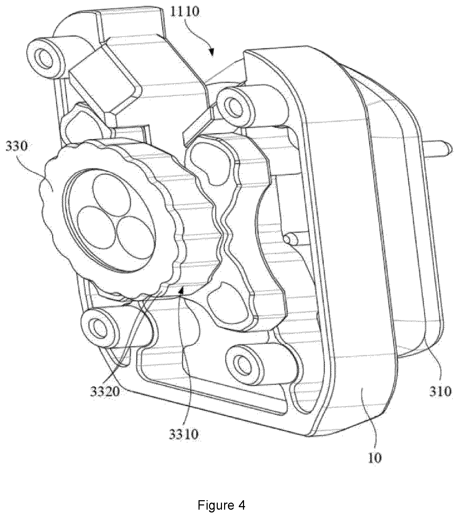

FIG. 4 is a schematic diagram showing the assembly of the fixing base and the locking block according to the embodiment of the invention;
FIG. 5 is a schematic view showing a structure of a vertical quick clamping fixture with a mounting portion removed in accordance with an embodiment of the present invention;
FIG. 6 is a schematic diagram showing a cross-sectional structure at A-A of FIG. 1 in accordance with an embodiment of the present invention;

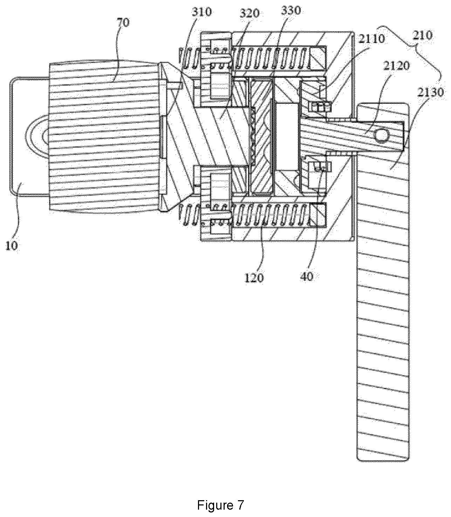

FIG. 7 is a schematic diagram showing a cross-sectional structure at B-B in FIG. 1 in accordance with an embodiment of the present invention;
FIG. 8 is a schematic view showing the overall structure of a stopper in an embodiment of the present invention;
FIG. 9 is a schematic view showing another angle of the stopper in the embodiment of the present invention;
Fig. 10 is a schematic structural view of the fixing portion and the limiting member in the embodiment of the present invention.

### DETAILED DESCRIPTION

In order to enable those skilled in the art to better understand the present application, the following description will make clear and complete descriptions of the technical solutions in the embodiments of the present application with reference to the accompanying drawings in the embodiments of the present application. It will be apparent that the described embodiments are merely some, but not all, of the embodiments of the present application. All other embodiments, which can be made by one of ordinary skill in the art based on the embodiments herein without making any inventive effort, shall fall within the scope of the present application.

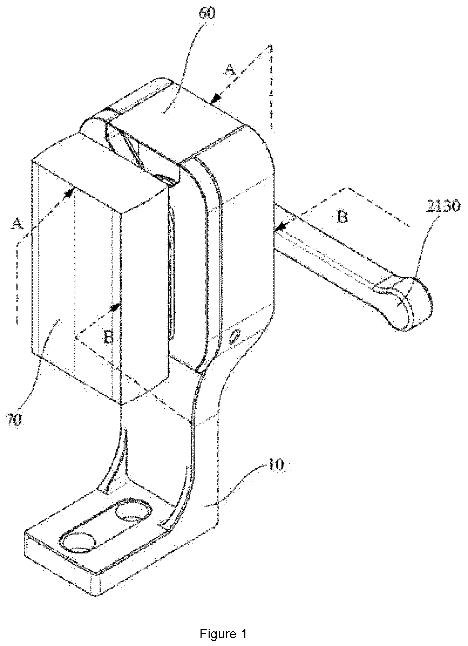

An embodiment of the present invention provides a vertical quick clamping and fixing device, please refer to Figures 1-3, including:
A holder 10 including a mounting slot 110 having an upper end opening 1110;
The locking block 30 comprises a mounting part 310, a connecting part 320 and a fixing part 330, wherein the mounting part 310 is used for fixedly mounting with the to-be-fixed piece 70, and the connecting part 320 is used for connecting the mounting part 310 and the fixing part 330;
the connection part 320 can extend into the mounting groove 110 from the opening 1110 to place the fixing part 330 in the fixing base 10, and the connection part 320 can also rotate in the mounting groove 110;
the edge of the fixing portion 330 is provided with a limiting groove 3310 at equal intervals, two sides of the limiting groove 3310 are provided with guide surfaces 3320, the fixing base 10 further comprises an elastic positioning portion 120, the elastic positioning portion 120 is elastic and can stretch out and draw back, so that the elastic positioning portion 120 is always abutted to the edge of the fixing portion 330, and the elastic positioning portion 120 can move in or out of the limiting groove 3310 along the guide surfaces 3320.

Compared with the prior art, the vertical quick clamping and fixing device of the invention firstly fixes the fixing seat 10, and fixedly connects the mounting part 310 of the locking block 30 with the pump body in advance. When the pump body needs to be mounted or replaced, the locking block 30 with the pump body is directly inserted into the mounting groove 110 from the opening 1110. Then, the elastic positioning portion 120 abuts against the edge of the fixing portion 10, and since the edge has a natural guiding surface and a limiting groove 3310, the elastic positioning portion 120 can be always located in the limiting groove 3310. When the position of the pump body needs to be adjusted, the pump body is directly rotated to drive the fixing part 10 to rotate. The elastic positioning portion 120 is elastic and can expand and contract, so that the elastic positioning portion can move from one limiting groove 3310 to the other limiting groove 3310, and the purpose of repositioning the pump body is achieved.

In general, for better effect, the elastic positioning portion 120 may be provided in plurality, preferably in two and symmetrically.

Specifically, the elastic positioning part can be an elastic glass bead or a pin shaft with a built-in compression spring.

The fixing base 10 is generally provided with a mounting hole, through which a bolt, a screw, or the like may be inserted to fix the fixing base 10.

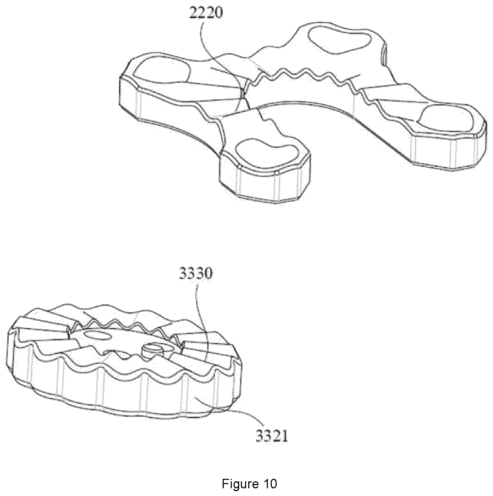

In addition, the form of the fixing portion 330 may be various. Referring to Fig. 10, a regular continuous wavy edge may be provided at the edge of the fixing portion 330. Of course, the guide surfaces 3320 in Fig. 10 are connected to each other, and in practice, the guide surfaces 3320 may be provided without being connected to each other, and may be specifically provided according to actual needs. The guide surfaces 3320 may be inclined surfaces or curved surfaces, and in some special cases, the adjacent bottom portions of the two guide surfaces 3320 automatically form the limiting grooves 3310.

In this embodiment, the connection portion 320 can be rotated circumferentially in the mounting groove 110.

In this application, the pump body is illustrated as the fastener 70.

In a preferred embodiment, the device further comprises a locking mechanism (not shown), the locking mechanism (not shown) comprises a rotating member 210 and a limiting member 220 which are in abutting connection with each other, the rotating member 210 is rotatably mounted on the fixed seat 10, the limiting member 220 is movably arranged on the fixed seat 10, and the rotating member 210 can enable the limiting member 220 to move along the axial direction of the rotating member 210.
the fixing portion 330 can be pressed by the stopper 220 to fix the locking block 30 to the fixing base 10.

In this embodiment, after the locking block 30 is inserted into the mounting groove 110 from the opening 1110, the rotating member 210 is rotated to move the limiting member 220 along the axial direction of the rotating member 210, and then the limiting member 220 and the fixing base 10 cooperate to clamp the fixing portion 330 of the locking block 30, so as to achieve the purpose of quick and convenient mounting and fixing of the pump body.

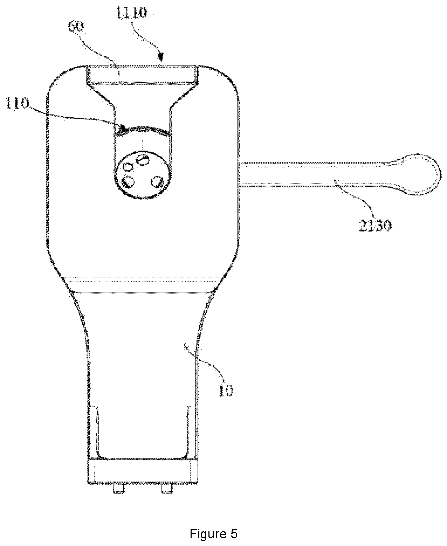

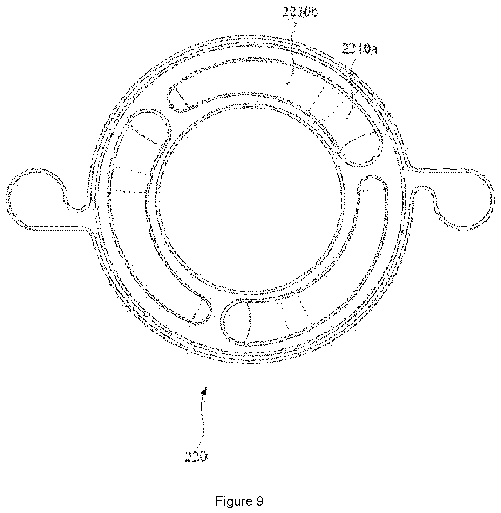

In a preferred embodiment, referring to Figures 5 and 9, the rotating member 210 includes a rotating shaft 2110 and a rotating cam 2120, the rotating cam 2120 is fixedly disposed at one end of the rotating shaft 2110 near the fixing base 10, and a protrusion (not shown) is disposed on a side of the rotating cam 2120 opposite to the rotating shaft 2110; the stopper 220 is provided at a side facing the rotator 210 with an arc groove 2210 engaged with a protrusion (not shown), and the height of the arc groove 2210 is set from high to low in the circumferential direction of the rotator 210.

In this embodiment, the shaft 2110 is driven to rotate the rotational cam 2120, and the protrusions on the rotational cam 2120 move in the arcuate slots 2210 on the limiter 220. Because the height of the arc groove 2210 is set from high to low in the circumferential direction of the rotation member 210, the rotation protrusion (not shown) along with the rotation cam 2120 starts to push the stopper 220 to move in the axial direction of the rotation shaft 2110, thereby pressing the fixing portion 330 of the locking block 30. Thus, an operator can realize the fixed installation of the pump body by only rotating the rotating shaft 2110.

In general, in order to maintain balance, a plurality of protrusions are generally provided, and the plurality of protrusions are uniformly spaced in the axial direction of the rotational cam 2120. Correspondingly, the arc-shaped groove 2210 on the limiting piece 220 is correspondingly arranged.

It should be noted that, in the initial state, the protrusion is generally located at the highest position of the arc-shaped groove 2210, so as to leave enough space for the fixing portion 330 of the locking block 30 to enter into the fixing base 10.

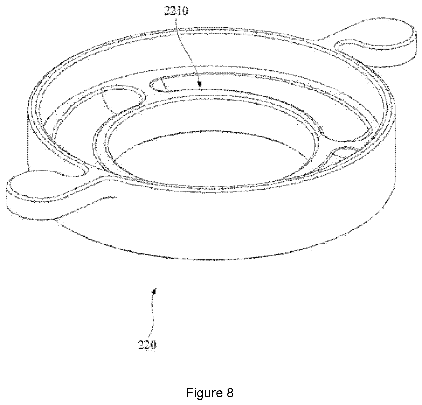

In a more preferred embodiment, the arcuate slot 2210 is provided with a first segment 2210a and a second segment 2210b, the first segment 2210a having a greater inclination of the slot bottom than the second segment 2210 b. In this embodiment, referring to Figures 8 and 9, the first segment 2210a of the arcuate slot 2210 is higher than the second segment 2210 b. Before rotating the shaft 2110, the protrusion is located at the lowest position of the first segment 2210 a. After rotating the shaft 2110, the protrusion moves the limiter 220 rapidly in the first segment 2210 a. After entering the second segment 2210b, the speed of movement of the stop member 220 is slowed. The first segment 2210a is provided with a groove bottom inclination angle larger than that of the second segment 2210b because the second segment 2210b has a lower groove bottom inclination angle to exert a certain locking effect. The stopper 220 is not easily pushed back by the fixing portion 330 to reversely rotate the rotational cam 2120, so that the pump body starts to loosen. It is also an object to reduce the travel by reducing the self-locking angle by this arrangement.

In a preferred embodiment, the rotating member 210 further includes a grip 2130, the grip 2130 being actuated to rotate the shaft 2110. The purpose of the grip 2130 is to drive the rotation of the shaft 2110 in an aspect to achieve the purpose of manually installing the pump body.

In a more preferred embodiment, a first wave surface 2220 is disposed on a side of the limiting member 220 facing the fixing portion 330, and a second wave surface 3330 is disposed on a side of the fixing portion 330 facing the limiting member 220, where the first wave surface 2220 and the second wave surface 3330 can be engaged. In this embodiment, the first wave surface 2220 and the second wave surface 3330 can be engaged, so that the stopper 220 and the fixing portion 330 are fixed to each other, so as not to cause relative rotation between the stopper 220 and the fixing portion 330, thereby shaking the pump body.

The wavy surface serves as a guide to enable smoother engagement or disengagement between the stopper 220 and the fixing portion 330. Of course, other embodiments are possible to achieve the relative fixation of the stop member 220 and the fixation portion 330. For example, the opposite surfaces of the stopper 220 and the fixing portion 330 are provided with anti-slip patterns, by which friction between the stopper 220 and the fixing portion 330 is increased.

In a more preferred embodiment, referring to Fig. 10, the limiting groove 3310 and the guiding surface 3320 are arranged end to form a third waveform surface 3321, and the trough of the second waveform surface 3330 corresponds to the crest of the third waveform surface, and the crest of the second waveform surface 3330 corresponds to the trough of the third waveform surface 3321. In this embodiment, the pump body is heavy, so the center of gravity of the entire lock block 30 is outside the fixing base 10. Under the action of gravity, one surface of the fixing portion 330 is abutted against the inner wall of the fixing base 10, so as to provide a certain damping effect.

On the fixing portion 330, the wave trough of the second wave surface 3330 corresponds to the wave crest at the edge, and the wave crest of the second wave surface 3330 corresponds to the wave trough at the edge, so that the elastic positioning portion 120 just abuts against the wave trough at the wave-shaped edge of the fixing portion 330 when the first wave surface 2220 and the second wave surface 3330 are engaged. Avoiding the possible failure of the engagement of the first and second undulating surfaces 2220, 3330 or failure of the resilient positioning portion 120.

In addition, under the action of gravity, part of the first waveform surface 2220 is also abutted against part of the second waveform surface 3330, so that a damping sense is further provided. The lock block 30 can be prevented from rotating until the first and second wave surfaces 2220 and 3330 are not fully engaged.

It should be noted that the first wave surface 2220 is generally a separate component, which forms a part of the limiter 220. The reason for this is to reduce the difficulty of machining the stopper 220.

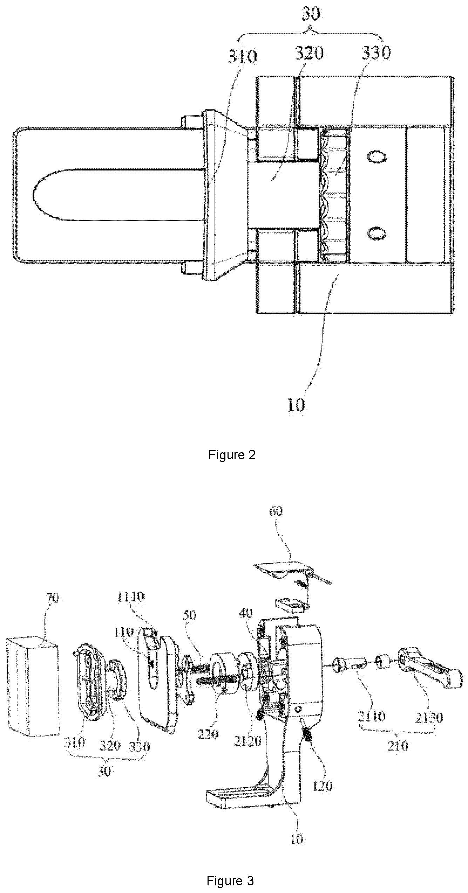

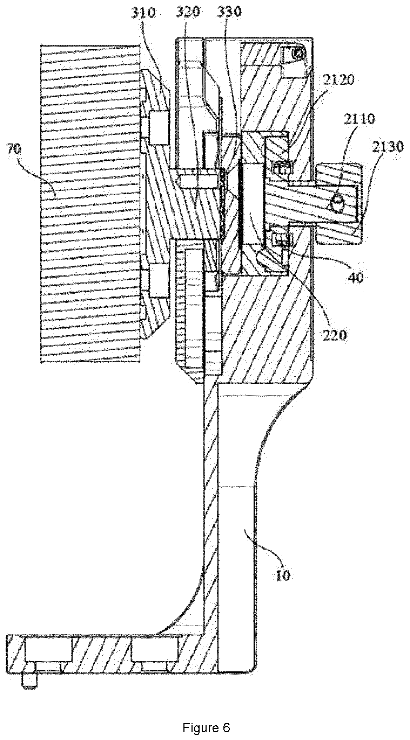

In a preferred embodiment, please refer to Fig. 3 and 6, the device further includes a first elastic member 40, wherein the first elastic member 40 is respectively connected to the fixing base 10 and the rotating cam 2120, so that the rotating cam 2120 can be reset after being rotated, and in this embodiment, the first elastic member 40 is a torsion spring. In this embodiment, when the pump body needs to be disassembled, the shaft 2110 is rotated, and only a small actuating force is required to separate the limiting member 220 and the fixing portion 330 under the action of the first elastic member 40.

Note that, since the force of rotation of the rotational cam 2120 is a torque force, the direction of the force of the stopper 220 to the rotational cam 2120 is perpendicular to the direction of the torque force. Therefore, it does not generally happen that the stopper 220 is pressed by the fixing portion 330 to turn the rotational cam 2120. Also, the friction between the stopper 220 and the rotational cam 2120 overcomes the elastic force of the first elastic member 40, thereby ensuring that the fixed pump body is not loosened after being mounted.

In a more preferred embodiment, referring to Fig. 6, the fixing device further includes a second elastic member 50, where the second elastic member 50 is disposed between the fixing base 10 and the limiting member 220, and the second elastic member 50 can keep the limiting member 220 away from the fixing portion 330. In this embodiment, when the pump body needs to be released, the second elastic member 50 can pull the limiting member 220 away from the fixing portion 330, so that the power-assisted limiting member 220 releases the clamping of the fixing portion 330, and the locking block 30 can be taken out from the fixing base 10.

In a preferred embodiment, the connecting portion 320 is cylindrical so that the locking block 30 can be rotated. In this embodiment, the connecting portion 320 is cylindrical, so that the whole locking block 30 can be rotated to a certain angle and then fixed by the limiting member 220 and the fixing portion 330, thereby enabling the pump body to reach a desired position.

Generally, the bottom of the mounting groove 110 is generally semicircular or arcuate in shape to fit the cylindrical connecting portion 320. And, the length of the connection portion 320 is generally equal to or slightly greater than the thickness of the mounting groove 110, and the diameter of the connection portion 320 is also equal to or slightly less than the width of the mounting groove 110. This prevents the connection between the locking block 30 and the fixing base 10 from shaking.

In a preferred embodiment, please refer to Fig. 1 and 5, further comprising a dust-proof structure 60, wherein the dust-proof structure 60 can be hinged to the fixing base 10 and can cover the opening 1110. The dust-proof structure 60 can relatively seal the entire holder 10, thereby reducing dust and risk of infection for the patient.

Specifically, the dust-proof structure 60 may be formed of a flip plate and a hinge, by which the flip plate is hinged to the hinge, thereby achieving the purpose of opening the opening 1110 and closing the opening 1110. Alternatively, the dust-proof structure 60 may be moved on a slide plate on a predetermined track on the holder 10. Eventually achieving the purpose of opening the opening 1110 and closing the opening 1110.

The vertical quick clamping fixture in all the above examples has the following specific embodiments:
1. firstly, fixedly connecting a pump body with an installation part 310 of a locking block 30, and fixing a fixed seat 10 at a certain place;
2. the connecting part 320 of the locking block 30 is put into the mounting groove 110 from the opening 1110, so that the fixing part 330 is placed in the fixing base 10;
3. rotating the pump body to adjust the position of the pump body, the elastic positioning part 120 is propped against the edge of the fixing part 330 and finally falls into the limiting groove 3310 so as to fix the position of the pump body;
4. the holding piece 2130 is driven to rotate the rotating shaft 2110, so that the rotating cam 2120 is rotated, the protrusion on the rotating cam 2120 moves in the arc-shaped groove 2210 of the limiting piece 220, and the limiting piece 220 is pushed to move towards the fixing portion 330 and is matched with the inner wall of the fixing seat 10 to clamp the fixing portion 330;
5. when the pump body is disassembled, the holding piece 2130 is reversely driven, the rotary cam 2120 rotates under the action of the first elastic piece 40, the second elastic piece 50 drives the limiting piece 220 to be separated from the fixing portion 330, and finally the pump body and the locking block 30 are taken out of the fixing seat 10.

Finally, it should be noted that: the above embodiments are only for illustrating the technical solution of the present application, and not for limiting the same; although the present application has been described in detail with reference to the foregoing embodiments, it should be understood by those of ordinary skill in the art that: the technical scheme described in the foregoing embodiments can be modified or some or all of the technical features thereof can be replaced by equivalents; such modifications and substitutions do not depart from the spirit of the corresponding technical solutions from the scope of the technical solutions of the embodiments of the present application.

## Claims

1. A vertical quick clamp fixture comprising:
a fixing seat including a mounting groove with an upper end opening;
a locking block including a mounting part, a connecting part and a fixing part, wherein the mounting part is used for being fixedly mounted with a piece to be fixed, and the connecting part is used for connecting the mounting part and the fixing part;
wherein the connecting part can extend into the mounting groove from the opening so as to place the fixing part in the fixing seat, and the connecting part can also rotate in the mounting groove;
wherein an edge of the fixing part is provided with limiting grooves at equal intervals, the two sides of the limiting grooves are provided with guide surfaces, the fixing seat further comprises elastic positioning parts which are elastic and can stretch out and draw back, so that the elastic positioning parts are always abutted against the edge of the fixing part, and the elastic positioning parts can move in or out of the limiting grooves along the guide surfaces;
a locking mechanism includes a limiting piece and a rotating piece, the rotating piece is in mutual abutting connection with the limiting piece, the limiting piece is movably arranged on the fixed seat, the rotating piece is rotatably arranged on the fixed seat, the rotating piece can be rotated to enable the limiting piece to move along the axial direction of the rotating piece, and the fixed part can be extruded by the limiting piece to enable the locking piece to be fixed on the fixed seat; and
a first wave surface is arranged on one side of the limiting part facing the fixed part, a second wave surface is arranged on one side of the fixed part facing the limiting part, and the first wave surface and the second wave surface can be matched;
wherein when the to-be-fixed piece is mounted on the locking block, the gravity center of the locking block is outside the fixed seat, and under the action of gravity of the to-be-fixed piece, part of the first wavy surface can be abutted with part of the second wavy surface.

2. The vertical quick clamping fixture of claim 1 wherein the rotating member comprises a shaft and a rotating cam, the rotating cam is fixedly disposed at one end of the shaft adjacent to the fixed seat, and a protrusion is disposed on a side of the rotating cam facing away from the shaft; the limiting piece is provided with an arc-shaped groove matched with the protrusion on one side facing the rotating piece, and the height of the arc-shaped groove is set from high to low along the circumferential direction of the rotating piece.

3. The vertical quick clamp fixture of claim 2 wherein said arc-shaped groove has a first section and a second section, said first section having a groove bottom angle of inclination greater than a groove bottom angle of inclination of said second section.

4. The vertical quick clamp fixture of any of the preceding claims wherein said rotatable member further comprises a grip member, said grip member being actuated to rotate said spindle.

5. The vertical quick clamp fixture of any of the preceding claims, wherein the limit groove and the guide surface are arranged end to form a third wave surface, the wave trough of the second wave surface corresponds to the wave crest of the third wave surface, and the wave crest of the second wave surface corresponds to the wave trough of the third wave surface.

6. The vertical quick clamp fixture of claim 3 further comprising a first resilient member connecting the anchor block and the rotating cam respectively to allow the rotating cam to return after being rotated.

7. The vertical quick clamp fixture of claim 6 further comprising a second resilient member disposed between said anchor mount and said stop member, said second resilient member being capable of moving said stop member away from said anchor portion.

8. The vertical quick clamp fixture of any of the preceding claims wherein said connecting portion is cylindrical to facilitate rotation of said locking block.

9. The vertical quick clamp fixture of any of the preceding claims further comprising a dust guard capable of articulating to said mounting base and capable of covering said opening.

10. A vertical quick clamp fixture comprising:
a fixing seat including a mounting groove with an upper end opening;
a locking block including a mounting part, a connecting part and a fixing part, wherein the mounting part is used for being fixedly mounted with a piece to be fixed, and the connecting part is used for connecting the mounting part and the fixing part;
wherein the connecting part can extend into the mounting groove from the opening so as to place the fixing part in the fixing seat, and the connecting part can also rotate in the mounting groove; and
a locking mechanism includes a limiting piece and a rotating piece, the rotating piece is in mutual abutting connection with the limiting piece, the limiting piece is movably arranged on the fixed seat, the rotating piece is rotatably arranged on the fixed seat, the rotating piece can be rotated to enable the limiting piece to move along the axial direction of the rotating piece, and the fixed part can be extruded by the limiting piece to enable the locking piece to be fixed on the fixed seat.

11. The fixture of claim 10 wherein an edge of the fixing part is provided with limiting grooves at equal intervals, the two sides of the limiting grooves are provided with guide surfaces, the fixing seat further comprises elastic positioning parts which are elastic and can stretch out and draw back, so that the elastic positioning parts are always abutted against the edge of the fixing part, and the elastic positioning parts can move in or out of the limiting grooves along the guide surfaces;

12. The fixture of claims 10 or 11 further comprising a first wave surface arranged on one side of the limiting part facing the fixed part, a second wave surface is arranged on one side of the fixed part facing the limiting part, and the first wave surface and the second wave surface can be matched.

13. The fixture of any of claims 10 to 12 wherein when the to-be-fixed piece is mounted on the locking block, the gravity center of the locking block is outside the fixed seat, and under the action of gravity of the to-be-fixed piece, part of the first wavy surface can be abutted with part of the second wavy surface.
